# FASCICULE DE BREVET EUROPEEN

(11) **EP 3 464 171 B1**
(45) Date de publication et mention de la délivrance du brevet: **01.07.2020**
(21) Numéro de dépôt: 17733492.7
(22) Date de dépôt: 24.05.2017
(51) Int. Cl.: C01B 3/02, C01B 3/52, C01B 3/50, F25J 3/02, C01B 32/40

(54) **PROCÉDÉ ET INSTALLATION POUR LA PRODUCTION COMBINÉE D'UN MÉLANGE D'HYDROGÈNE ET D'AZOTE AINSI QUE DE MONOXYDE DE CARBONE PAR DISTILLATION ET LAVAGE CRYOGÉNIQUES**
VERFAHREN UND ANLAGE ZUR KOMBINIERTEN HERSTELLUNG EINER MISCHUNG AUS WASSERSTOFF UND STICKSTOFF SOWIE KOHLENMONOXID DURCH KRYOGENE DESTILLATION UND WASCHEN
METHOD AND FACILITY FOR COMBINED PRODUCTION OF A MIXTURE OF HYDROGEN AND NITROGEN AS WELL AS CARBON MONOXIDE BY CRYOGENIC DISTILLATION AND SCRUBBING

(30) Priorité: 06.06.2016 FR 1655118
(43) Date de publication de la demande: 10.04.2019
(73) Titulaire: L'Air Liquide Société Anonyme pour l'Etude et l'Exploitation des Procédés Georges Claude, 75007 Paris (FR)
(72) Inventeur: DEMOLLIENS, Bertrand, 75011 Paris (FR)
(74) Mandataire: Mercey, Fiona Susan
(86) Numéro de dépôt international: PCT/FR2017/051281
(87) Numéro de publication internationale: WO 2017/212136

(56) Documents cités:
- EP-A1- 0 937 681
- EP-A1- 1 245 533
- EP-A2- 0 317 851
- EP-A2- 0 928 937
- WO-A2-2008/087318
- BRIAN A MC NEIL AIR PRODUCTS AND CHEMICALS ET AL: "Process and apparatus for separation of nitrogen from carbon monoxide (Brian A. McNeil, Air Products and Chemicals, Inc.)", RESEARCH DISCLOSURE, MASON PUBLICATIONS, HAMPSHIRE, GB, vol. 426, no. 54, 1 octobre 1999 (1999-10-01), XP007124911, ISSN: 0374-4353

## Description

La présente invention est relative à un procédé et installation pour la production combinée d'un mélange d'hydrogène et d'azote, constituant éventuellement un gaz de synthèse d'ammoniac ainsi que de monoxyde de carbone et éventuellement de méthane par distillation et lavage cryogéniques

Il est connu d'utiliser des procédés de type lavage au méthane, décrits dans EP 0 465 366, pour séparer un gaz de synthèse en ses différents constituants. Ce procédé repose notamment sur l'utilisation d'une fraction d'un fluide riche en méthane récupéré en cuve de colonne CO/CH4 comme fluide de lavage des deux premières colonnes, l'autre fraction de ce fluide étant alors récupérée sous forme de purge de méthane.

Par équilibre thermodynamique entre le méthane de lavage et le gaz de tête de colonne, une partie du méthane est alors perdue dans le gaz de tête des deux colonnes. Le lavage de la colonne de stripping pour augmenter le rendement est décrit dans EP 0 317 851.

Il faudra donc à tout moment avoir une quantité de méthane suffisante dans le gaz à traiter pour compenser ces pertes de méthane. Si le gaz de synthèse à traiter ne contient pas assez de méthane, on ne pourra pas utiliser ce procédé tel quel. Il sera donc nécessaire d'utiliser un appoint de gaz riche en méthane pour augmenter artificiellement le taux de méthane dans le gaz entrant à traiter afin de pouvoir procéder à un lavage au méthane tel que décrit dans DE 37 41 906 A1.

Utiliser un procédé de condensation partielle réduit considérablement le rendement d'extraction de CO par rapport à un lavage au méthane. Il est nécessaire dans ce cas de consommer plus de « carburant » en amont (gaz naturel, naphta, charbon... etc) pour produire le gaz de synthèse afin d'obtenir une même production de monoxyde de carbone.

Il est également connu d'utiliser de traiter un gaz riche en hydrogène par de l'azote liquide dans une colonne de lavage pour créer un mélange de gaz nécessaire à la synthèse de l'ammoniac, ayant une stœchiométrie aux alentours de 3H2 pour 1N2.

Certains documents, tel que EP 0 937 681 A1 abordent déjà la coproduction de CO et de gaz pour l'ammoniac. Néanmoins, leur principal défaut est de :
- Soit réduire le rendement d'extraction CO (on perd typiquement environ 8-10% de CO dans la purge liquide au milieu de colonne de lavage)
- Soit polluer le produit CO avec de l'azote (Une colonne supplémentaire et de l'énergie sont alors nécessaires pour re-séparer ce mélange...)

L'objet de l'invention est de coupler la production de monoxyde de carbone avec celle d'un mélange pour la production d'ammoniac. Un tronçon supplémentaire est rajouté en tête de colonne de lavage afin de laver successivement le gaz de synthèse avec (de haut en bas) de l'azote et du méthane. L'azote ajouté ne se retrouvera pas dans le produit CO. Néanmoins l'azote présent dans le gaz de synthèse à l'entrée se retrouvera à la sortie dans le produit CO, dans le cas où il n'y a pas de colonne de déazotation. On pourra rajouter également une colonne de strippage qui permettra de récupérer une portion du méthane qui autrement aurait été perdu avec le gaz en tête de colonne de lavage. Cette colonne de strippage peut être constituée par une enceinte avec un seul plateau théorique, par exemple un pot séparateur ou une enceinte permettant une séparation équivalente à plusieurs plateaux théoriques.

La récupération supplémentaire de ce méthane peut permettre :
D'utiliser un procédé de lavage au méthane avec des quantités faibles de méthane dans le gaz de synthèse Typiquement, le gaz de synthèse produit par un ATR est assez pauvre en méthane, étant aux environs de 1% molaire...

Une teneur de méthane inférieure à 0.4% molaire dans le gaz de synthèse à l'entrée devient suffisante pour effectuer un lavage au méthane.
- Autrement:
   ∘ Il aurait fallu soit faire un procédé de condensation partielle avec une perte importante de rendement CO
   ∘ Il aurait fallu importer un gaz supplémentaire G1 pour augmenter artificiellement le taux de méthane dans le gaz entrant à traiter afin de pouvoir procéder à un lavage au méthane tel que décrit dans DE 37 41 906 A1. On traite donc plus de gaz pour une même production de CO, ce qui rend nécessaires des équipements plus gros et/ou une énergie consommée plus importante : ceci peut occasionner une importation potentielle d'impuretés qui existe dans le gaz G1 et non dans le gaz de synthèse qu'il faudra alors gérer.
   ∘ Il est également possible de procéder à un recyclage interne de méthane tel que décrit dans EP 0 790 212 A1 ce qui permet d'aider également
- À récupérer plus de méthane pour pouvoir ensuite le produire liquide ou gazeux.

Coupler ces deux procédés permet également de n'utiliser qu'une seule boîte froide au lieu de deux boîtes froides et également de réduire la taille de la turbine CO par rapport à un lavage au méthane classique et donc de réduire le débit de cycle CO (et donc de consommer moins d'énergie). Effectivement, le mélange d'azote et d'hydrogène est endothermique, ce qui permet de créer du froid par un autre moyen que la turbine de CO.

Selon un objet de l'invention, il est prévu un procédé pour la production combinée d'un mélange d'hydrogène et d'azote, de monoxyde de carbone et éventuellement de méthane et éventuellement d'azote par distillation et lavage cryogéniques dans lequel :
i) on refroidit un mélange gazeux contenant au moins de l'hydrogène, du monoxyde de carbone et du méthane et éventuellement de l'azote dans un échangeur de chaleur
ii) on envoie le mélange refroidi à une colonne de lavage
iii) on introduit un liquide riche en méthane à un premier niveau intermédiaire de la colonne de lavage comme premier liquide de lavage
iv) on introduit au moins un liquide riche en azote à un niveau supérieur au premier niveau de la colonne de lavage comme deuxième liquide de lavage
v) on soutire un mélange d'hydrogène et d'azote comme gaz de tète de la colonne de lavage
vi) on soutire un liquide de cuve de la colonne de lavage et on l'envoie à une colonne de strippage,
vii) on soutire un liquide au niveau d'une section intermédiaire de la colonne de lavage que l'on envoie soit vers l'échangeur de chaleur, soit vers une conduite de gaz de tête de la colonne de strippage, soit vers une deuxième colonne de strippage.
viii) on soutire un liquide de cuve de la colonne de strippage et on l'envoie à une colonne de séparation de monoxyde de carbone et de méthane,
ix) au moins une partie du liquide de cuve de la colonne de séparation constitue le liquide de l'étape iii) et
x) on soutire un fluide riche en monoxyde de carbone de la colonne de séparation.

Selon d'autres aspects facultatifs de l'invention :
- le procédé est maintenu au froid au moins partiellement par détente d'au moins une partie du fluide riche en monoxyde de carbone ou d'au moins une partie du gaz de tête de la colonne de strippage dans une turbine.
- le procédé est maintenu au froid au moins partiellement par mélange du gaz de tête de la colonne de lavage avec un liquide riche en azote provenant d'une source extérieure à une température intermédiaire de l'échangeur de chaleur et/ou à la température du bout froid de l'échangeur de chaleur.
- la colonne de lavage, la colonne de strippage et la colonne de séparation sont dans la même enceinte isolée.
- le mélange gazeux contient au plus 0.4% molaires de méthane et constitue la seule source de méthane pour le procédé ni de recyclage interne de méthane.
- on récupère une partie du liquide de cuve de la colonne de séparation comme produit riche en méthane sous forme liquide ou sous forme gazeuse, après l'avoir vaporisé dans l'échangeur de chaleur.
- le liquide riche en azote de l'étape iv) provient d'une source extérieure et n'est pas contenu dans le mélange gazeux en amont de l'échangeur de chaleur.
- le liquide riche en azote de l'étape iv) ne provient pas d'une source extérieure et est contenu dans le mélange gazeux en amont de l'échangeur de chaleur.
- un premier liquide riche en azote de l'étape iv) provient d'une source extérieure et n'est pas contenu dans le mélange gazeux en amont de l'échangeur de chaleur et un deuxième liquide riche en azote de l'étape iv) ne provient pas d'une source extérieure et est contenu dans le mélange gazeux en amont de l'échangeur de chaleur.
- l'au moins un liquide riche en azote est envoyé en tête de la colonne de lavage.
- on refroidit un gaz soutiré d'une région intermédiaire de la colonne de lavage avec un liquide riche en monoxyde de carbone soutiré de la colonne de séparation et on renvoie le gaz refroidi à la colonne de lavage.
- le gaz de tête de la colonne de lavage contient au moins cinq, voire au moins neuf parts d'hydrogène pour une part d'azote et éventuellement est enrichi en azote pour atteindre les trois parts d'hydrogène.
- le mélange gazeux contient de l'azote et au moins une partie du liquide riche en azote envoyé à la colonne de lavage provient d'une colonne de déazotation qui épure le fluide riche en monoxyde de carbone en azote.
- le liquide riche en méthane produit contient au moins un autre hydrocarbure plus lourd, tel que de l'éthane.

Selon un autre objet de l'invention, il est prévu une installation pour la production combinée d'un mélange d'hydrogène et d'azote, de monoxyde de carbone et de méthane et éventuellement d'azote par distillation et lavage cryogéniques comprenant un échangeur de chaleur, une colonne de lavage, une colonne de strippage, une colonne de séparation de monoxyde de carbone et de méthane, une conduite pour envoyer un mélange gazeux contenant au moins de l'hydrogène, du monoxyde de carbone et du méthane se refroidir dans l'échangeur de chaleur (7), une conduite pour envoyer le mélange refroidi de l'échangeur de chaleur à la colonne de lavage, une conduite pour introduire un liquide riche en méthane à un premier niveau intermédiaire de la colonne de lavage comme premier liquide de lavage, au moins une conduite pour introduire un liquide riche en azote à un niveau supérieur au premier niveau de la colonne de lavage comme deuxième liquide de lavage, une conduite pour soutirer un mélange d'hydrogène et d'azote comme gaz de tête de la colonne de lavage, une conduite pour soutirer un liquide de cuve de la colonne de lavage et pour envoyer le liquide de cuve de la colonne de lavage à la colonne de strippage, une conduite pour soutirer un liquide au niveau d'une section intermédiaire de la colonne de lavage et pour l'envoyer soit vers l'échangeur de chaleur, soit vers une conduite de gaz de tête de la colonne de strippage, soit vers une deuxième colonne de strippage, une conduite pour soutirer un liquide de cuve de la colonne de strippage et l'envoyer à la colonne de séparation de monoxyde de carbone et de méthane, au moins une partie du liquide de cuve de la colonne de séparation constituant le liquide riche en méthane et une conduite pour soutirer un fluide riche en monoxyde de carbone de la colonne de séparation.

Le principe de l'invention est, à partir du procédé de lavage au méthane, de rajouter un tronçon en tête de colonne de lavage. Le premier tronçon (du bas) de la colonne de lavage traite le gaz de synthèse par du méthane liquide de façon classique. Le gaz continuera ensuite vers le deuxième tronçon. Avant de faire intégrer le deuxième tronçon, on pourra refroidir le gaz à -181°C avec du CO liquide pour récupérer du méthane qui se condensera (d'une manière analogue à celle décrite dans FR 2 807 505). Le liquide de cuve de colonne de lavage est classiquement envoyé à une colonne de strippage puis à la colonne CO/CH4.

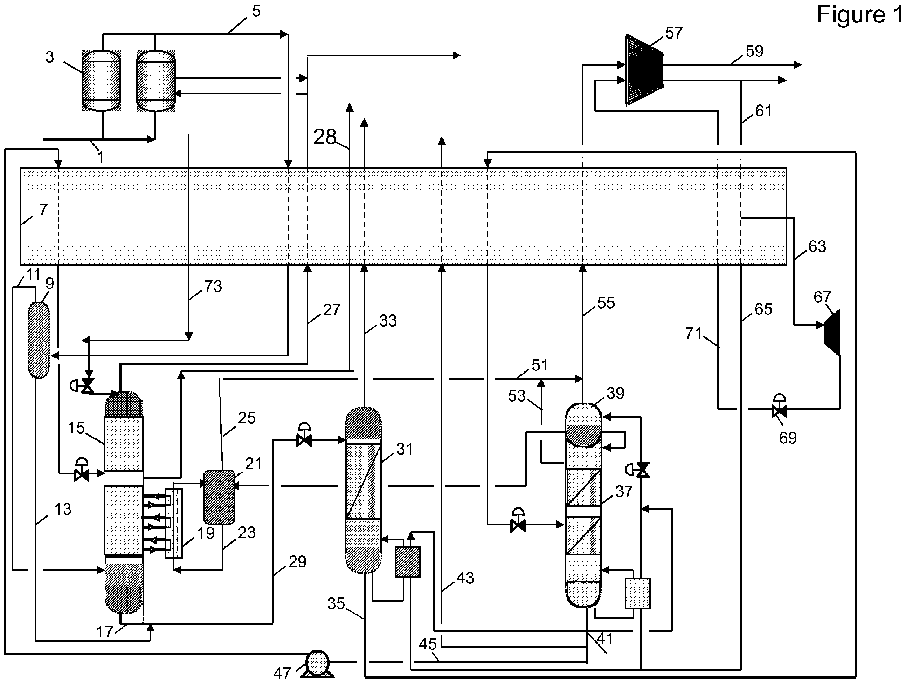

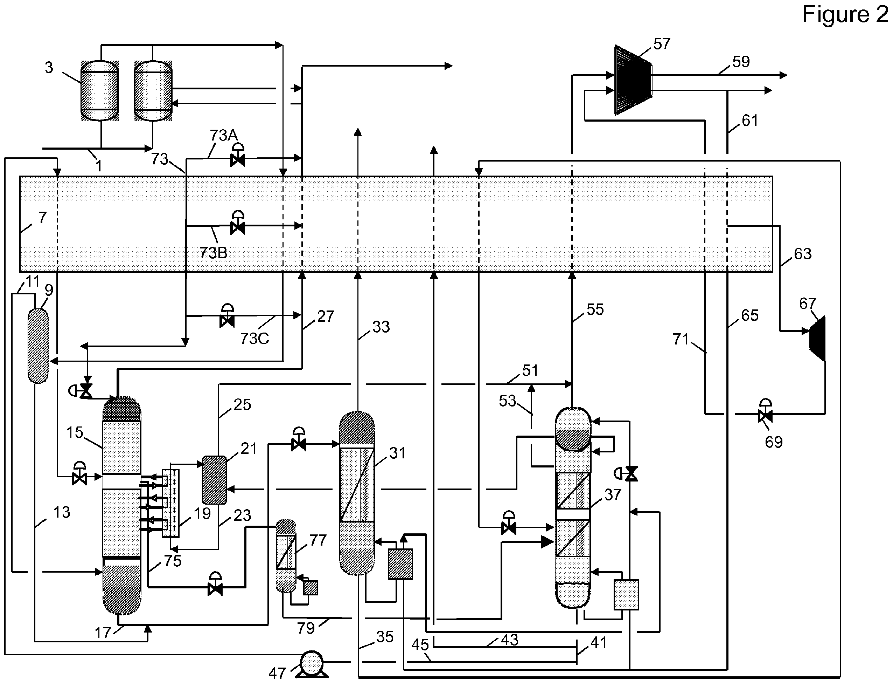

Dans le deuxième tronçon (du haut), on lave le gaz avec de l'azote. Le liquide de bas de ce deuxième tronçon, sera ensuite récupéré et soit vaporisé directement (Figure 1), soit envoyé vers une deuxième colonne de strippage (Figure 2). On opérera cette deuxième colonne de strippage à une pression de 3 bars environ. On réchauffera la cuve de cette deuxième colonne de strippage afin de réduire la concentration en azote du liquide pour ne pas polluer le produit CO avec de l'azote. On enverra le liquide de la cuve de cette colonne dans la colonne CO/CH4. On récupère au passage une petite fraction de CO (environ 0.1% du débit de CO entrant) ce qui permet également d'augmenter le rendement CO. Le gaz de tête de la deuxième colonne de strippage sera réchauffé dans l'échangeur principal puis purgé.

Le gaz en tête de colonne de lavage ne contenant donc plus de méthane après le lavage à l'azote, est soit envoyé directement à l'échangeur 7, soit envoyé dans un vaporiseur-condenseur pour générer du CO liquide à partir de LP CO. (En effet, le mélange N2/H2 est endothermique et la température de ce gaz est de -193°C environ.)

On pourra mélanger l'azote nécessaire à l'ajustement du rapport H2/N2
i) directement à froid (c'est-à-dire sans réchauffage du gaz de tête de colonne de lavage riche en hydrogène dans l'échangeur, ou au bout froid) et/ou
ii) à un niveau intermédiaire de l'échangeur (c'est-à-dire après réchauffage partiel du gaz de tête de colonne de lavage dans l'échangeur principal)

Ces deux solutions permettant de produire du froid et donc de réduire l'appoint de froid par la turbine.

Il est également possible d'effectuer le mélange à chaud, c'est-à-dire en aval de l'échangeur principal, ce qui permet généralement de réduire l'investissement.

On pourra envoyer ce mélange de gaz de la même façon que la tête de colonne soit directement à l'échangeur 7, soit envoyé dans un vaporiseur-condenseur pour générer du CO liquide à partir de LP CO, soit utilisé pour refroidir la section de colonne de lavage au méthane d'une façon analogue à FR 2 807 504.

On peut également ajouter de l'azote partiellement à froid ou à un niveau intermédiaire de l'échangeur et procéder au mélange final pour atteindre la stoechiométrie désirée à chaud. L'invention sera décrite en plus de détail en se référant aux trois figures qui illustrent des procédés selon l'invention.

Selon le procédé de la Figure 1, un débit de gaz de synthèse contenant au moins de l'hydrogène, du monoxyde de carbone et du méthane 1 est épuré en eau, dioxyde de carbone, méthanol et autres impuretés dans l'unité de purification 3 et ensuite le débit 5 de gaz de synthèse sec se refroidit dans l'échangeur de chaleur 7, qui est de préférence un échangeur à plaques en aluminium brasé. Le gaz refroidi est envoyé à un séparateur de phases 9 et le gaz formé 11 est envoyé en bas d'une colonne de lavage 15. La colonne de lavage est alimentée à un niveau intermédiaire, appelé premier niveau intermédiaire de la colonne de lavage 15, avec un liquide riche en méthane 45 comme premier liquide de lavage. On introduit un liquide riche en azote 73 à un niveau supérieur au premier niveau de la colonne de lavage 15 comme deuxième liquide de lavage. On soutire un mélange d'hydrogène et d'azote 27 comme gaz de tête de la colonne de lavage 15 et on soutire un liquide de cuve de la colonne de lavage. Le gaz de tête 27 de la colonne de lavage contient au moins cinq, voire au moins neuf parts d'hydrogène pour une part d'azote et est enrichi en azote pour atteindre les trois parts d'hydrogène.

Le liquide de cuve 17 de la colonne de lavage est mélangé avec le liquide 13 du séparateur de phases 9 et le mélange 29 est envoyé en tête d'une colonne de strippage 31 ; Un liquide intermédiaire 28 est soutiré au premier niveau intermédiaire de la colonne de lavage 51 et envoyé se vaporiser dans l'échangeur de chaleur 7. Sans ce soutirage, le produit riche en monoxyde de carbone serait pollué en azote Un liquide de cuve 35 est soutiré de la colonne de strippage 31 et envoyé à une colonne de séparation de monoxyde de carbone et de méthane 37 à un niveau intermédiaire après refroidissement dans l'échangeur de chaleur 7, Au moins une partie 45 du liquide de cuve 41 de la colonne de séparation 37 constitue le premier liquide de lavage après pressurisation dans la pompe 47. Un fluide riche en monoxyde de carbone 53 est soutiré en tête de la colonne de séparation 37. La colonne de séparation 37 comprend un stockage de monoxyde de carbone liquide 39 en tête de colonne. Le gaz 53 et le gaz du stockage sont envoyés comme gaz 55 à un compresseur 57 de monoxyde de carbone pour fournir le produit riche en monoxyde de carbone 59. Une partie 61 du monoxyde de carbone est refroidie dans l'échangeur de chaleur 7 et divisée en deux ; une partie 63 est détendue dans une turbine 67 et une vanne 69 pour être réchauffée et renvoyée au compresseur 57. Le reste 65 sert à rebouillir les colonnes 31 et 37 et à alimenter le stockage 39.

Du monoxyde de carbone liquide 49 du stockage 39 sert à refroidir des fluides intermédiaires de la colonne de lavage à un point en dessous de l'arrivée du premier liquide de lavage. L'enceinte 21 recueille le liquide et du liquide 23 est envoyé vers un échangeur 19 pour refroidir les débits intermédiaires. Le gaz 25 de l'enceinte est renvoyé au compresseur 57.

Un gaz de flash 33 sort de la tête de la colonne de strippage 31 et un produit riche en méthane 43 est éventuellement soutiré de la colonne CO/CH4 37 sous forme liquide et vaporisé dans l'échangeur 7.

Le procédé de la Figure 2 diffère de celui de la Figure 1 en ce qu'une deuxième colonne de strippage 77 a été rajoutée, opérant à environ 3 bars. Alimentée en tête par un liquide pris en dessus de l'arrivée du premier liquide de lavage, elle est chauffée par le cycle de monoxyde de carbone. Le liquide de cuve 79 est envoyé à la colonne CO/CH4 37 au même niveau que le débit 35.

Pour arriver au rapport requis d'hydrogène et d'azote, on peut rajouter de l'azote de la source externe au gaz de tête de la colonne de lavage en amont de l'échangeur de chaleur et/ou à un niveau intermédiaire de l'échangeur de chaleur et/ou en aval de l'échangeur de chaleur.

Il sera compris dans les exemples des deux figures que le gaz de tête de la colonne de lavage peut contenir de l'hydrogène et de l'azote dans un rapport 3 :1 tel que requis pour un gaz de synthèse d'ammoniac ou pas. Dans le cas où l'azote présent serait insuffisant, de l'azote peut être rajouté à la sortie de la colonne comme illustré pour la Figure 2 afin d'atteindre le rapport requis.

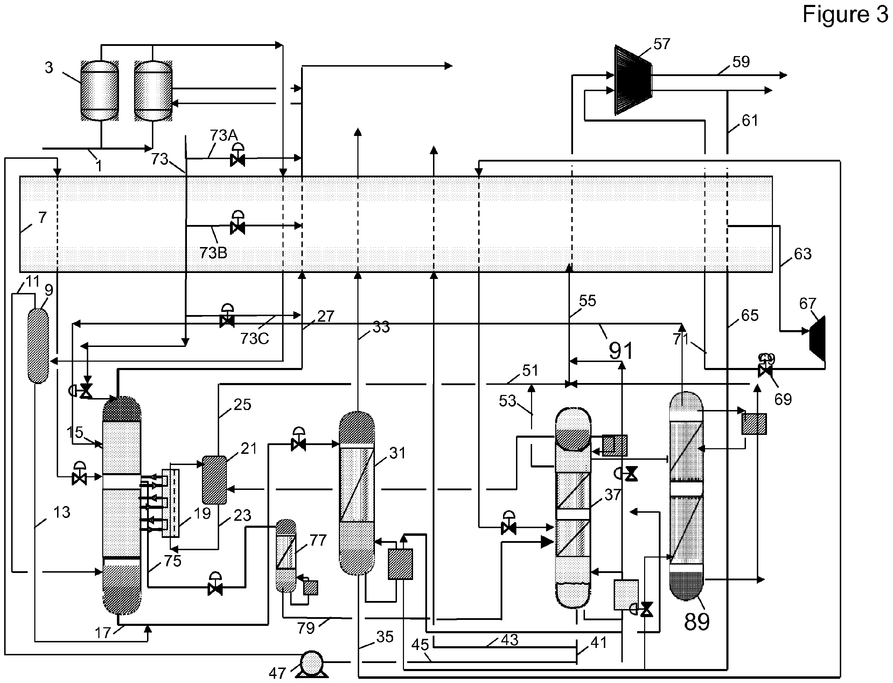

Dans la Figure 3, une colonne de déazotation 89 a été rajoutée. Alimentée en cuve par du monoxyde de carbone 65 provenant du compresseur 57, elle produit du monoxyde de carbone dépourvu d'azote en cuve qui alimente le compresseur 57. Le débit riche en azote produit en tête de la colonne 89 est détendu, liquéfié et envoyé en tête de la colonne 15 comme liquide de lavage. Dans ce cas, le débit 73 peut être réduit, voire aucun apport d'azote autre que le gaz d'alimentation 1 n'est nécessaire. Dans l'exemplaire, le liquide 91 est introduit dans la colonne 15 en dessous de l'arrivée du liquide 73.

## Revendications

1. Procédé pour la production combinée d'un mélange d'hydrogène et d'azote, de monoxyde de carbone et éventuellement de méthane par distillation et lavage cryogéniques dans lequel :
i) on refroidit un mélange gazeux (1) contenant au moins de l'hydrogène, du monoxyde de carbone et du méthane et éventuellement de l'azote dans un échangeur de chaleur (7),
ii) on envoie le mélange refroidi à une colonne de lavage (15),
iii) on introduit un liquide riche en méthane (45) à un premier niveau intermédiaire de la colonne de lavage comme premier liquide de lavage,
iv) on introduit au moins un liquide riche en azote (73, 91) à un niveau supérieur au premier niveau de la colonne de lavage comme deuxième liquide de lavage,
v) on soutire un mélange d'hydrogène et d'azote comme gaz de tête (27) de la colonne de lavage,
vi) on soutire un liquide de cuve (17) de la colonne de lavage et on l'envoie à une colonne de strippage (31),
vii) on soutire un liquide (28, 75) au niveau d'une section intermédiaire de la colonne de lavage que l'on envoie soit vers l'échangeur de chaleur, soit vers une conduite de gaz de tête de la colonne de strippage, soit vers une deuxième colonne de strippage,
viii) on soutire un liquide de cuve (35) de la colonne de strippage et on l'envoie à une colonne de séparation (37) de monoxyde de carbone et de méthane,
ix) au moins une partie du liquide de cuve (41,45) de la colonne de séparation constitue le liquide de l'étape iii), et
x) on soutire un fluide (53) riche en monoxyde de carbone de la colonne de séparation.

2. Procédé selon la revendication 1 dans lequel le procédé est maintenu au froid
i) au moins partiellement par détente d'au moins une partie du fluide riche en monoxyde de carbone ou d'au moins une partie du gaz de tête de la colonne de strippage dans une turbine (67) et/ou
ii) au moins partiellement par mélange du gaz de tête de la colonne de lavage avec un liquide riche en azote (73A,73B,73C) provenant d'une source extérieure à une température intermédiaire de l'échangeur de chaleur (7) et/ou à la température du bout froid et/ou chaud de l'échangeur de chaleur

3. Procédé selon l'une des revendications précédentes dans lequel la colonne de lavage (15), la colonne de strippage (31) et la colonne de séparation (37) sont dans la même enceinte isolée.

4. Procédé selon l'une des revendications précédentes dans lequel le mélange gazeux (1) contient au plus 0.4% molaires de méthane et constitue la seule source de méthane pour le procédé.

5. Procédé selon l'une des revendications précédentes dans lequel on récupère une partie (43) du liquide de cuve de la colonne de séparation comme produit riche en méthane sous forme liquide ou sous forme gazeuse, après l'avoir vaporisé dans l'échangeur de chaleur (7).

6. Procédé selon l'une des revendications précédentes dans lequel le liquide riche en azote (73) de l'étape iv) provient d'une source extérieure et n'est pas contenu dans le mélange gazeux en amont de l'échangeur de chaleur (7).

7. Procédé selon l'une des revendications précédentes dans lequel le liquide riche en azote (73,91) est envoyé en tête de la colonne de lavage.

8. Procédé selon l'une des revendications précédentes dans lequel on refroidit un gaz soutiré d'une région intermédiaire de la colonne de lavage avec un liquide riche en monoxyde de carbone soutiré de la colonne de séparation et on renvoie le gaz refroidi à la colonne de lavage.

9. Procédé selon l'une des revendications précédentes dans lequel le gaz de tête (27) de la colonne de lavage contient au moins cinq, voire au moins neuf parts d'hydrogène pour une part d'azote et éventuellement est enrichi en azote pour atteindre les trois parts d'hydrogène.

10. Installation pour la production combinée d'un mélange d'hydrogène et d'azote, de monoxyde de carbone et éventuellement de méthane et éventuellement de l'azote par distillation cryogénique comprenant un échangeur de chaleur (7), une colonne de lavage (15), une colonne de strippage (31), une colonne de séparation de monoxyde de carbone et de méthane (37), une conduite pour envoyer un mélange gazeux (1) contenant au moins de l'hydrogène, du monoxyde de carbone et du méthane se refroidir dans l'échangeur de chaleur (7), une conduite pour envoyer le mélange refroidi de l'échangeur de chaleur à la colonne de lavage, une conduite pour introduire un liquide riche en méthane à un premier niveau intermédiaire de la colonne de lavage comme premier liquide de lavage, une conduite pour introduire un liquide riche en azote à un niveau supérieur au premier niveau de la colonne de lavage comme deuxième liquide de lavage, une conduite pour soutirer un mélange d'hydrogène et d'azote comme gaz de tête de la colonne de lavage, une conduite pour soutirer un liquide de cuve de la colonne de lavage et pour envoyer le liquide de cuve de la colonne de lavage à la colonne de strippage, une conduite pour soutirer un liquide (28,75) au niveau d'une section intermédiaire de la colonne de lavage et pour l'envoyer soit vers l'échangeur de chaleur, soit vers une conduite de gaz de tête de la colonne de strippage, soit vers une deuxième colonne de strippage (77), une conduite pour soutirer un liquide de cuve de la colonne de strippage et l'envoyer à la colonne de séparation de monoxyde de carbone et de méthane, au moins une partie du liquide de cuve de la colonne de séparation constituant le liquide riche en méthane et une conduite pour soutirer un fluide riche en monoxyde de carbone de la colonne de séparation.

## Patentansprüche

1. Verfahren zur kombinierten Herstellung eines Gemisches aus Wasserstoff und Stickstoff, Kohlenmonoxid und eventuell Methan durch kryogene Destillation und Waschen, wobei:
i) ein Gasgemisch (1), enthaltend mindestens Wasserstoff, Kohlenmonoxid und Methan und eventuell Stickstoff, in einem Wärmeaustauscher (7) abgekühlt wird,
ii) das abgekühlte Gemisch in eine Waschkolonne (15) geschickt wird,
iii) eine methanreiche Flüssigkeit (45) in ein erstes Zwischenniveau der Waschkolonne als erste Waschflüssigkeit eingeführt wird,
iv) mindestens eine stickstoffreiche Flüssigkeit (73, 91) in ein höheres Niveau als das erste Niveau der Waschkolonne als zweite Waschflüssigkeit eingeführt wird,
v) ein Gemisch aus Wasserstoff und Stickstoff als Kopfgas (27) von der Waschkolonne abgezogen wird,
vi) eine Sumpfflüssigkeit (17) von der Waschkolonne abgezogen und sie an eine Stripping-Kolonne (31) geschickt wird,
vii) eine Flüssigkeit (28, 75) auf dem Niveau eines Zwischenabschnitts von der Waschkolonne abgezogen wird, die entweder zum Wärmeaustauscher oder zu einer Leitung von Kopfgas der Stripping-Kolonne oder zu einer zweiten Stripping-Kolonne geschickt wird,
viii) eine Sumpfflüssigkeit (35) von der Stripping-Kolonne abgezogen wird und sie an eine Trennkolonne (37) von Kohlenmonoxid und Methan geschickt wird,
ix) mindestens ein Teil der Sumpfflüssigkeit (41,45) der Trennkolonne die Flüssigkeit von Schritt iii) darstellt, und
x) ein kohlenmonoxidreiches Fluid (53) von der Trennkolonne abgezogen wird.

2. Verfahren nach Anspruch 1, wobei das Verfahren kalt gehalten wird
i) mindestens teilweise durch Entspannung mindestens eines Teils des kohlenmonoxidreichen Fluids oder mindestens eines Teils des Kopfgases der Stripping-Kolonne in einer Turbine (67) und/oder
ii) mindestens teilweise durch Mischen des Kopfgases der Waschkolonne mit einer stickstoffreichen Flüssigkeit (73A,73B,73C), die aus einer äußeren Quelle stammt, bei einer Zwischentemperatur des Wärmeaustauschers (7) und/oder bei der Temperatur des kalten und/oder warmen Endes des Wärmeaustauschers.

3. Verfahren nach einem der vorstehenden Ansprüche, wobei sich die Waschkolonne (15), die Stripping-Kolonne (31) und die Trennkolonne (37) im gleichen isolierten Gefäß befinden.

4. Verfahren nach einem der vorstehenden Ansprüche, wobei das Gasgemisch (1) höchstens 0,4 Molprozent Methan enthält und die einzige Methanquelle für das Verfahren darstellt.

5. Verfahren nach einem der vorstehenden Ansprüche, wobei ein Teil (43) der Sumpfflüssigkeit der Trennkolonne als methanreiches Produkt in flüssiger Form oder in gasförmiger Form wiedergewonnen wird, nachdem er im Wärmeaustauscher (7) verdampft wurde.

6. Verfahren nach einem der vorstehenden Ansprüche, wobei die stickstoffreiche Flüssigkeit (73) von Schritt iv) aus einer äußeren Quelle stammt und nicht in dem Gasgemisch vorgelagert vom Wärmeaustauscher (7) enthalten ist.

7. Verfahren nach einem der vorstehenden Ansprüche, wobei die stickstoffreiche Flüssigkeit (73,91) an den Kopf der Waschkolonne geschickt wird.

8. Verfahren nach einem der vorstehenden Ansprüche, wobei ein Gas, abgezogen von einer Zwischenregion der Waschkolonne, mit einer kohlenmonoxidreichen Flüssigkeit abgekühlt wird, die von der Trennkolonne abgezogen ist, und das abgekühlte Gas an die Waschkolonne zurückgeschickt wird.

9. Verfahren nach einem der vorstehenden Ansprüche, wobei das Kopfgas (27) der Waschkolonne mindestens fünf, sogar mindestens neun Teile Wasserstoff für einen Teil Stickstoff enthält und eventuell mit Stickstoff angereichert ist, um die drei Teile Wasserstoff zu erreichen.

10. Anlage zur kombinierten Herstellung eines Gemisches aus Wasserstoff und Stickstoff, Kohlenmonoxid und eventuell Methan und eventuell Stickstoff durch kryogene Destillation, umfassend einen Wärmeaustauscher (7), eine Waschkolonne (15), eine Stripping-Kolonne (31), eine Trennkolonne von Kohlenmonoxid und von Methan (37), eine Leitung, um ein Gasgemisch (1), das mindestens Wasserstoff, Kohlenmonoxid und Methan enthält, zum Abkühlen im Wärmeaustauscher (7) zu schicken, eine Leitung, um das abgekühlte Gemisch des Wärmeaustauschers an die Waschkolonne zu schicken, eine Leitung, um eine methanreiche Flüssigkeit in ein erstes Zwischenniveau der Waschkolonne als erste Waschflüssigkeit einzuführen, eine Leitung, um eine stickstoffreiche Flüssigkeit in ein höheres Niveau als das erste Niveau der Waschkolonne als zweite Waschflüssigkeit einzuführen, eine Leitung, um ein Gemisch von Wasserstoff und Stickstoff als Kopfgas von der Waschkolonne abzuziehen, eine Leitung, um eine Sumpfflüssigkeit von der Waschkolonne abzuziehen und um die Sumpfflüssigkeit von der Waschkolonne an die Stripping-Kolonne zu schicken, eine Leitung, um eine Flüssigkeit (28,75) auf dem Niveau eines Zwischenabschnitts von der Waschkolonne abzuziehen, und um sie entweder an den Wärmeaustauscher oder an eine Leitung von Kopfgas der Stripping-Kolonne oder an eine zweite Stripping-Kolonne (77) zu schicken, eine Leitung, um eine Sumpfflüssigkeit von der Stripping-Kolonne abzuziehen und sie an die Trennkolonne von Kohlenmonoxid und Methan zu schicken, wobei mindestens ein Teil der Sumpfflüssigkeit der Trennkolonne die methanreiche Flüssigkeit darstellt, und eine Leitung, um ein kohlenmonoxidreiches Fluid von der Trennkolonne abzuziehen.

## Claims

1. Method for the combined production of a mixture of hydrocarbon and nitrogen, carbon monoxide and possibly methane by cryogenic distillation and scrubbing wherein:
i) a gaseous mixture (1) containing at least hydrogen, carbon monoxide and methane and possibly nitrogen is cooled in a heat exchanger (7),
ii) the cooled mixture is fed to a scrubbing column (15),
iii) a methane-rich liquid (45) is injected at a first intermediate level of a scrubbing column at a first intermediate level of a scrubbing column,
iv) at least one nitrogen-rich liquid (73, 91) is injected at a higher level than the first level of the scrub column as a second scrub liquid,
v) a mixture of hydrogen and nitrogen is extracted as an off-gas (27) from the scrubbing column,
vi) a sump liquid (17) is extracted from the scrubbing column and it is fed to a stripping column (31),
vii) a liquid (28, 75) is extracted at the level of an intermediate section of the scrubbing column that is fed either to the heat exchanger, or to a conduit of off-gas of the stripping column, or to a second stripping column,
viii) a sump liquid (35) is extracted from the stripping column and it is fed to a carbon monoxide and methane separation column (37),
ix) at least one portion of the sump liquid (41, 45) from the separation column forms the liquid of step iii), and
x) a carbon monoxide-rich fluid (53) is extracted from the separation column.

2. Method according to claim 1, wherein the method is maintained cold
i) at least partially by the expansion of at least one portion of the carbon monoxide-rich fluid or of at least one portion of the off-gas from the stripping column in a turbine (67) and/or
ii) at least partially by mixing the off-gas from the scrubbing column with a nitrogen-rich liquid (73A, 73B, 73C) coming from an outside source at an intermediate temperature of the heat exchanger (7) and/or at the temperature of the cold and/or hot end of the heat exchanger.

3. Method according to one of the preceding claims, wherein the scrubbing column (15), the stripping column (31) and the separation column (37) are in the same insulated enclosure.

4. Method according to one of the preceding claims, wherein the gaseous mixture (1) contains at most 0.4 % mol of methane and forms the only source of methane for the method.

5. Method according to one of the preceding claims, wherein a portion (43) of the sump liquid from the separation column is recovered as a methane-rich product in liquid form or in gaseous form, after having vaporised it in the heat exchanger (7).

6. Method according to one of the preceding claims, wherein the nitrogen-rich liquid (73) of step iv) comes from an outside source and is not contained in the gaseous mixture upstream of the heat exchanger (7).

7. Method according to one of the preceding claims, wherein the nitrogen-rich liquid (73, 91) is fed at the head of the scrubbing column.

8. Method according to one of the preceding claims, wherein a gas extracted from an intermediate region of the scrubbing column is cooled with a liquid rich in carbon monoxide extracted from the separation column and the cooled gas is returned to the scrubbing column.

9. Method according to one of the preceding claims, wherein the off-gas (27) from the scrubbing column contains at least five, even at least nine parts hydrogen for one part nitrogen and possibly is enriched with nitrogen to reach three parts hydrogen.

10. Installation for the combined production of a mixture of hydrogen and nitrogen, carbon monoxide and possibly methane and possibly nitrogen by cryogenic distillation comprising a heat exchanger (7), a scrubbing column (15), a stripping column (31), a carbon monoxide and methane separation column (37), a conduit for feeding a gaseous mixture (1) containing at least hydrogen, carbon monoxide and methane to be cooled in the heat exchanger (7), a conduit for feeding the cooled mixture from the heat exchanger to the scrubbing column, a conduit for injecting a methane-rich liquid at a first intermediate level of a scrubbing column as a first scrub liquid, a conduit for injecting a nitrogen-rich liquid at a higher level than the first level of the scrub column as a second scrub liquid, a conduit for extracting a mixture of hydrogen and nitrogen as an off-gas from the scrubbing column, a conduit for extracting a sump liquid from the scrubbing column and for feeding the sump liquid from the scrubbing column to the stripping column, a conduit for extracting a liquid (28, 75) at the level of an intermediate section from the scrubbing column and for feeding it either to the heat exchanger, or to a conduit of off-gas from the stripping column, or to a second stripping column (77), a conduit for extracting a sump liquid from the stripping column and feeding it to the carbon monoxide and methane separation column, at least one portion of the sump liquid from the separation column forming the methane-rich liquid and a conduit for extracting a carbon monoxide-rich fluid from the separation column.
